# EUROPEAN PATENT APPLICATION

(11) **EP 1 731 514 A1**
(43) Date of publication of application: **13.12.2006**
(21) Application number: 05104809.8
(22) Date of filing: 02.06.2005
(51) Int. Cl.: C07D 277/82

(54) **Process for the preparation of Pramipexole**

(71) Applicant: SANDOZ AG, 4056 Basel (CH)
(72) Inventor: Bendre, Deven Sharad, 400-607, Kolshet, Thane (IN); Holkar, Anil Ganpat, 400-607, Kolshet, Thane (IN); Kantikar, Gajanan Tammanna, 400 607, Kolshet, Thane (IN)
(74) Representative: Dietz, Jörg-Reimar

(57) **Abstract**

The present invention relates to a new process for the preparation of 2,6-diamino-4,5,6,7-tetrahydobenzthiazole from 4-phtalimido cyclohexanone via an hydroiodide acid addition salt of 2-amino-6-phtalimido-4,5,6,7-terahydrobenzthiazole and to a novel hydroiodide acid addition salt of 2-amino-6-phtalimido-4,5,6,7-terahydrobenzthiazole.

## Description

The present invention relates to a new process for the preparation of 2,6-diamino-4,5,6,7-tetrahydobenzthiazole from 4-phtalimido cyclohexanone via an hydroiodide acid addition salt of 2-amino-6-phtalimido-4,5,6,7-terahydrobenzthiazole. More particularly the invention relates to a process for the preparation of pramipexole. The present invention also relates to a novel hydroiodide acid addition salt of 2-amino-6-phtalimido-4,5,6,7-terahydrobenzthiazole.

(S)-4,5,6,7-tetrahydro-N⁶-propyl-2,6-benzothiazolediamine (or (S)-2-amino-4,5,6,7-tetrahydro -6-(propylamino)benzotriazole) of formula (I) and its pharmaceutical acceptable salts known as pramipexole, are useful in treating disorders related to Parkinson's disease.

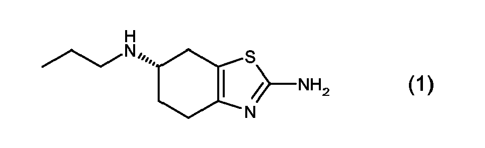

2,6-diamino-4,5,6,7-tetrahydobenzthiazole of formula (2) is the key intermediate in most of the processes for the preparation of pramipexole.

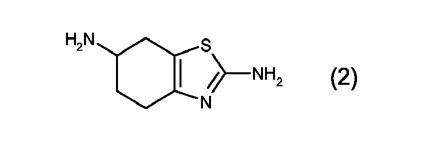

For example, EP0186087 generically describes a manufacturing process wherein a ring halogenation (preferably bromination) of a substituted aminoketone (3) and the condensation of the obtained alpha-halogenaminoketone (4) with a thiourea to form a 2-aminotetrahydrobenzothiazole ring (2a) as illustrated by the following reaction scheme 1:

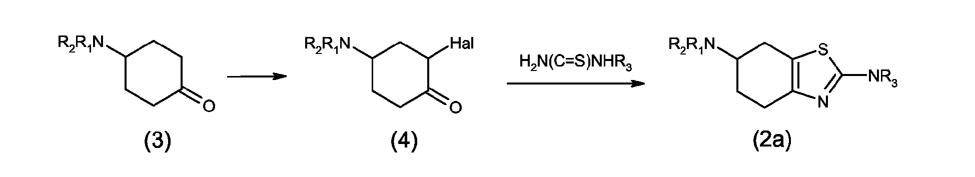

More specifically, EP0186087 describes processes for the preparation of 2,6-diamino-4,5,6,7-tetrahydobenzthiazole of formula (2). One of the described processes comprises the addition of a solution of bromine in glacial acetic acid to a mixture 4-(Phthalimido)-cyclohexanone of formula (5) and hydrobrominic acid in glacial acetic acid. The resulting mixture is stirred during several hours. The concentration of the reaction mixture in vacuo and treatment of the residue with diethylether gives intermediates 2-bromo-4-(phthalimido)-cyclohexanone of formula (6). Crude compound (6) is then treated with thiourea in ethanol under refluxing conditions to give 2-amino-6-phthalimindo-4,5,6,7-tetrahydrobenzothiazole of formula (7). Compound (2) is finally obtained after the treatment of compound (7) with hydrazine hydrate in ethanol. Compound (2) is extracted into chloroform and purified by chromatography. A yield of 13% is reported. This process can be summarized by the following scheme 2.

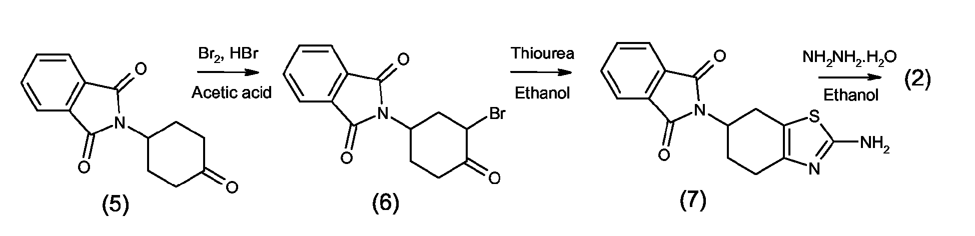

As mentioned above this manufacturing process as described in EP0186087 requires the use of bromine and hydrazine, which are hazardous and difficult to handle reagents, and involves a series of isolations and extractions followed by column purification. As a result this manufacturing process is not compatible with a scale up at an industrial scale.

A manufacturing process for the preparation of 2,6-diamino-4,5,6,7-tetrahydobenzthiazole of formula (2) is also disclosed by Schneider et al. in J. Med. Chem., vol. 30, N°. 3, pp. 494-498. According to this reference compound (2) is obtained from 4-acetamidocyclohexanone with a yield of 41%.

One can also mention WO2004/026850 describing a straight forward manufacturing process for the preparation of pramipexole wherein, 2,6-diamino-4,5,6,7-tetrahydobenzthiazole is not used as an intermediate though.

There is, therefore, a need for developing alternative processes for the industrial preparation of 2,6-diamino-4,5,6,7-tetrahydobenzthiazole of formula (2), with an improved yield, avoiding the use of hazardous and difficult to handle reagents.

The present invention provides a new process for the preparation of 2,6-diamino-4,5,6,7-tetrahydobenzthiazole from 4-phtalimido cyclohexanone via an hydroiodide acid addition salt of 2-amino-6-phtalimido-4,5,6,7-terahydrobenzthiazole.

This process avoids the drawback associated with the processes known from the prior art; i.e. use of bromine and hydrazine, and provide of 2,6-diamino-4,5,6,7-tetrahydobenzthiazole with an improved yield allowing it preparation at an industrial scale.

According to the present invention the Hydroiodide acid addition salt of 2-amino-6-phtalimido-4,5,6,7-terahydrobenzthiazole is directly isolated as a pure solid from reaction medium without any further purification to reach desired purity.

A first object of the present invention is related to a process for the preparation of 2-amino-6-phtalimido-4,5,6,7-terahydrobenzthiazole of formula (2) or a salt thereof 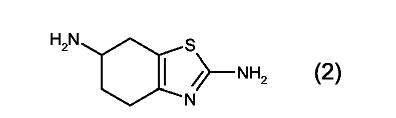 comprising the steps of:
- (a): reacting 4-phtalimido cyclohexanone of formula (5) with iodine and thiourea in an organic solvent or a mixture thereof to yield 2-Amino-6-phthalimido-4,5,6,7-terahydrobenzthiazole hydroiodide of formula (8) 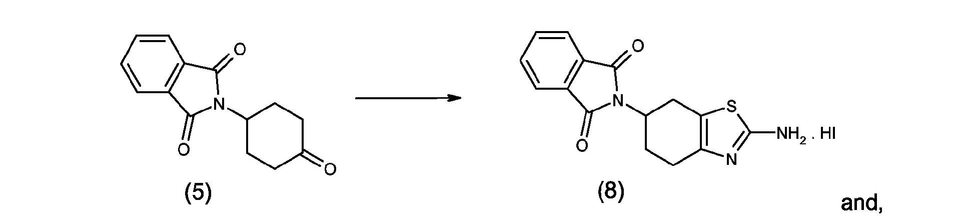
and,
- (b): removing the phthalimido group from compound (8) with a borohydride to yield 2-amino-6-phtalimido-4,5,6,7-terahydrobenzthiazole of formula (2) or a salt thereof.

Step (a) corresponds to an iodination and a cyclization process. The iodination is a very simple process in contrast to a bromination since iodine, as a solid, is much less dangerous to handle than bromine.

In step (a), the organic solvent is preferably a C1-C6 lower alkyl alcohol or a mixture thereof. More preferred C1-C6 alcohols include, but are not limited to, methanol, ethanol, 2-propanol (isopropanol), butanol (such as n-butanol, isobutanol, and t-butanol) and isoamyl alcohol (isopentanol) or a mixture thereof. Especially preferred C1-C6 alcohol is 2-propanol. From 8 to 25 volumes, especially from 13 to 15 volumes of organic solvent are used in step (a).

Preferably, from 1 to 2 equivalents, more preferably from 1.1 to 1.3 equivalents, of iodine and from 1.5 to 4, more preferably from 2.1 to 2.4 equivalents, of thiourea, are added in step (a).

Preferred process condition for step (a) after addition of all the reagents the solution mixture is stirred at 70 to 100°C, especially 80 to 85°C during 20 to 30 hours, especially 23 to 25 hours

Especially good yield and purity of compound (8) have been obtained when 2-propanole is used as organic solvent in step (a).

4-phtalimido cyclohexanone of formula (5) which is used as starting product in step (a) is obtainable, for example, by a manufacturing process as described in EP0186087.

A preferred process according to step (a) of the invention giving excellent results is the following:
- (a1): Adding 4-phtalimido cyclohexanone of formula (5) to iodine and thiourea in 2-propanol, especially at room temperature,
- (a2): refluxing the resulting solution, preferably with stirring during 24 hours,
- (a3): cooling the solution, especially to room temperature, filtering out the solid,
- (a4): adding water to the solid, filtering out the solid and,
- (a5): drying the solid of the desired compound (8).

The removal of the phthalimido group from 2-amino-6-phthalimido-4,5,6,7-tetrahydrobenzthiazole using of a borohydride according to step (b) is very smooth reaction, easily to be performed and resulting in high yields of 2,6-diamino-4,5,6,7-tetrahydobenzthiazole.

Borohydrides suitable for the removal of the phthalimido group according to step (b) are preferably alkali or earth alkali metal borohydrides. Particularly preferred borohydrides are selected from the group consisting of sodium, potassium or lithium borohydrides or mixtures thereof. Especially preferred borohydride is sodium borohydride.

Preferably from 2.5 to 5.0 equivalents, more preferably from 3.0 to 3.4 equivalents of borohydride are added in step (b).

Preferably in step (b) sodium borohydride is added, especially at room temperature, to a stirred suspension of 2-Amino-6-phthalimido-4,5,6,7-tetrahydrobenzothiazole hydroiodide in an organic solvent or a mixture thereof in the presence of water. The resulting suspension is then stirred preferably at a temperature from 30 to 80°C, preferably from 50 to 60°C, during a time from 1 to 10 hours, preferably from 1 to 3 hours. Thereafter, glacial acetic acid is added and the reaction mixture is heated at a temperature from 40 to 90°C, especially from 80 to 90°C, during a time from 2 to 12 hours, especially from 4 to 5 hours

The organic solvent used in step (b) is preferably a C1-C6 lower alkyl alcohol or a mixture thereof. More preferred C1-C6 alcohols include, but are not limited to, methanol, ethanol, 2-propanol (isopropanol), butanol (such as n-butanol, isobutanol, and t-butanol) and isoamyl alcohol (isopentanol) or a mixture thereof. Especially preferred C1-C6 alcohol is 2-propanol. From 10 to 25 volumes, especially from 14 to 16 volumes of organic solvent are used in step (b).

Preferably in step (b) the respective amount of 2-propanol, water and glacial acetic acid are from 10 to 25 volumes, especially from 14 to 16; from 2 to 5 volumes, especially from 2.8 to 3.2 volumes; and from 1.5 to 4 volumes, especially from 2.5 to 3 volumes.

A preferred process according to step (b) comprises the following sub-steps of :
- (b1): adding a borohydride, especially sodium borohydride, to a suspension of compound (8) in an organic solvent or a mixture thereof, especially 2-propanol, in the presence of water and,
- (b2): adding glacial acetic acid to the reaction mixture.

A more preferred process according to step (b) of the invention giving excellent results is the following:
- (b1'): Adding sodium borohydride, especially at room temperature, to a stirred suspension of 2-Amino-6-phthalimido-4,5,6,7-tetrahydrobenzothiazole hydroiodide in 2-Propanol and water,
- (b2'): stirring the resulting suspension, especially at 55-60°C for 2 hours,
- (b3'): adding glacial acetic acid,
- (b4'): heating the reaction to reflux, especially at 84-88°C for 5 hours,
- (b5'): distilling out 2-Propanol,
- (b6'): Adding water the reaction mixture and cooling the resulting solution, especially to room temperature and,
- (b7'): drying the solid of the desired compound (2) after an acid/base extraction of the reaction mixture.

For the purposes of this invention, a "salt" is any acid addition salt, preferably a pharmaceutically acceptable acid addition salt, including but not limited to a hydrohalogenic acid salt such as hydrofluoric, hydrochloric, hydrobromic and hydroiodic acid salt; an inorganic acid salt such as nitric, perchloric, sulfuric and phosphoric acid salt; an organic acid salt such as a sulfonic acid salt (for example methanesulfonic, trifluoromethanesulfonic, ethanesulfonic, isethionic, benzenesulfonic, p-toluenesulfonic or camphorsulfonic acid salt), acetic, malic, fumaric, succinic, citric, tartaric, benzoic, gluconic, lactic, mandelic, mucic, pamoic, pantothenic, oxalic and maleic acid salt; and an amino acid salt such as ornithinic, glutamic and aspartic acid salt. The acid addition salt may be a mono-or di-acid addition salt. A preferred salt is a di-hydrohalogenic,di-sulphuric, di-phosphoric or di-organic acid salt. A most preferred salt is a di-hydrochloric acid salt.

A second object of the present invention is related to a manufacturing process of pramipexole, comprising the step of forming 2,6-diamino-4,5,6,7-tetrahydrobenzothiazole according to the manufacturing process of the present invention as previously described, then converting it to pramipexole.
The conversion of 2,6-diamino-4,5,6,7-tetrahydrobenzothiazole to pramipexole is well known in the prior art, for example in EP0186087 and J. Med. Chem., vol. 30, N°. 3, pp. 494-498.

Both 2,6-diamino-4,5,6,7-tetrahydro-benzthiazole and pramipexole have an asymmetric carbon atom, and exist as two distinct enantiomers : the S(-) isomer and the R(+) isomer. The pharmacological activity of the S(-) isomer of pramipexole is, however, twice as high as that of the R(+) isomer, and the name pramipexole is commonly used to refer to the optically pure S(-) form. In this specification "2,6-diamino-4,5,6,7-tetrahydro-benzothiazole" encompasses the R(-) and S(-) enantiomers individually and also encompasses any mixture thereof including the racemic mixture, and the term "pramipexole" encompasses the R(+) and S(-) enantiomers individually and also the racemic mixture.

The resolution of a racemic mixture of 2,6-diamino-4,5,6,7-tetrahydro-benzthiazole can be carried out after step (b) described above. Methods of resolution are known in the art. Alternatively, pramipexole racemate can be produced prior to resolution, then the mixture resolved, if desired.
The resolution of racemic mixture of 2,6-diamino-4,5,6,7-tetrahydro-benzthiazole is described by Schneider et al. (J. Med. Chem. 30,494(1987)). This method uses 2,6-diamino-4,5,6,7-tetrahydro-benzthiazole as a substrate and L(+) tartaric acid as a resolution agent. After the resolution, optically active pramipexole can for example be prepared by two-step propylation of the single enantiomer of 2,6-diamino-4,5,6,7-tetrahydro-benzthiazole comprising the steps of reaction with propionic anhydride followed by a reduction of the propionyl intermediate.

A third object of the present invention is related to 2-Amino-6-phthalimido-4,5,6,7-terahydrobenzthiazole hydroiodide of formula (8).

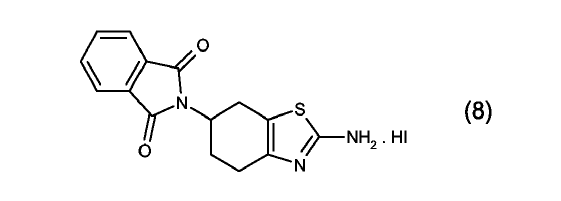

Compound of formula (8) is obtained from step (a) according to the manufacturing process of the present invention as previously described.
Accordingly a fourth object of the present invention is related to process for the preparation of formula (8) comprising reacting compound of formula (5) with iodine and thiourea.

Compound of formula (8), preferably prepared according to the present invention, can for example be used for the manufacture of pramipexole.

This invention will be better understood from the Examples that follow. However, these examples illustrate but do not limit the invention. Those skilled in the art will readily appreciate that the specific process and results discussed are merely illustrative of the invention as described more fully in the claims that follow thereafter.

The following non-limiting examples illustrate further aspects of the invention.

### Example 1

### Preparation of 2-Amino-6-phthalimido-4,5,6,7-tetrahydrobenzothiazole hydroiodide: (8)

1000 g of 4-(Phthalimido)cyclohexanone, 1150 g of Iodine, 690 g of Thiourea and 13 liters of 2-Propanol are added to the round bottom assembly at room temperature. The resulting solution is refluxed with stirring for 24 hours and then cooled to room temperature and filtered. The wet cake is stirred with 10 liters of water at room temperature for 1 hour. The solid is filtered, washed with 2 liters of water and dried in a vacuum oven for 8-10 hours at 65°C to give 1.55 kg of compound (8).
Yield: 92%
HPLC Purity : 96-98 %
Mp : 308-310°C

### Process for the purification of 2-Amino-6-phthalamido-4,5,6,7-tetrahydrobenzothiazole hydroiodide

Isopropyl alcohol (250 ml) is added to the crude 2-Amino-6-phthalamido-4,5,6,7-tetrahydrobenzothiazole hydroiodide (10 g) and stirred at reflux for 2 hours. The undissolved compound is filtered at 70°C. The wet compound obtained is dried at 60°C under vacuum for 2 hours to give 6.8 g of purified compound (8).

Analytical Data :

| | |
|---|---|
| Melting point: | 316.4°C |
| Water content: | 0.36% |
| lodine content: | 28.5% (% w/w) |

¹HNMR (DMSO-d6): ∂ 2.02 - 2.05 (m , 1H), 2.47 - 2.58 (m , 2H), 2.66 - 2.79 (m, 2H), 3.09 - 3.18 (m , 1H) , 4.40 - 4.80 (m , 1H), 7.84 - 7.91 (m , 4H , Aromatic) , 9.17 ( 2H , s , NH_{2.} exchanges with D₂O).
MASS (m/z) : 300 (M + 1)
IR (KBr) : 3298, 3232, 3281, 3090, 3030, 2852, 1767, 1716,1622, 1470, 1432, 1395, 1380, 1334, 1261, 1191, 1171, 1115, 1090, 1013, 886, 824, 802, 775, 718, 701, 637, 533, 508.

### Example 2

### Preparation of (±)-2,6-Diamino- 4,5,6,7-tetrahydrobenzothiazole: (2)

To a stirred suspension of 3000 g of 2-Amino-6-phthalimido-4,5,6,7-tetrahydrobenzothiazole hydroiodide in 45 liters of 2-Propanol and 9 liters of water are added 854 g of Sodium borohydride granules slowly and portion wise in a 100 liters glass reactor at room temperature. The suspension is then stirred at 55-60°C for 2 hours. 7.5 liters of glacial acetic acid is carefully added to avoid excessive foaming and the reaction mass is then heated to reflux at 84-88°C for 5 hours. 2-Propanol is distilled out under vacuum. 16 Liters of water is added to the reaction mass and the resulting solution is cooled to room temperature, acidified to pH 2 with 3.5 liters of concentrated HCl and washed with Dichloromethane (16 liters). The acidic aqueous layer is made alkaline (pH 12) by slowly adding 7.0 liters of 50% aqueous NaOH solution at 30-50°C. Reaction mass stirred at 28-32°C for 1 hour and the solid is filtered. Wet compound is dried under vacuum at 60-65°C for 8-10 hours to get 950-980 g of compound (2).
Yield: 88%
HPLC purity : 96-98 %
Mp : 223-225°C

## Claims

1. A process for the preparation of 2-amino-6-phtalimido-4,5,6,7-terahydrobenzthiazole of formula (2) or a salt thereof 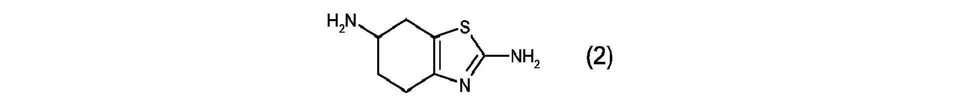 comprising the steps of:
(a) reacting 4-phtalimido cyclohexanone of formula (5) with iodine and thiourea in an organic solvent or a mixture thereof to yield 2-Amino-6-phthalimido-4,5,6,7-terahydrobenzthiazole hydroiodide of formula (8) 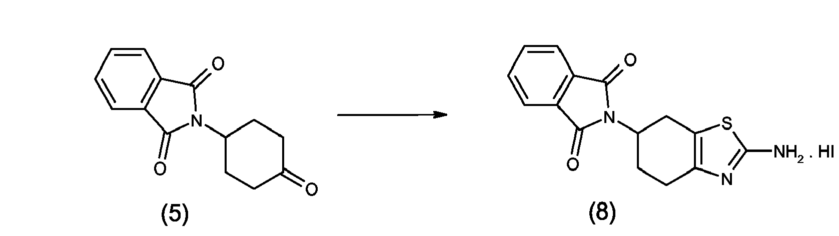
, and
(b) removing the phthalimido group from compound (8) with a borohydride to yield 2 amino-6-phtalimido-4,5,6,7-terahydrobenzthiazole of formula (2) or a salt thereof.

2. A process according to claim 1 wherein the solvent in step (a) is a C1-C6 lower alkyl alcohol or a mixture thereof.

3. A process according to claims 1 to 2, wherein the solvent in step (a) is 2-propanol.

4. A process according to claim 1 to 3, wherein step (b) comprises the sub-steps of
(b1) adding a borohydride to a suspension of compound (8) in an organic solvent or a mixture thereof in the presence of water and,
(b2) adding glacial acetic acid to the reaction mixture.

5. A process according to claims 1 to 4, wherein the organic solvent in step (b) is a C1-C6 lower alkyl alcohol or a mixture thereof and the borohydride is sodium borohydride.

6. A process according to claim 1 to 5 wherein the organic solvent in step (b) is 2-propanol.

7. A compound of formula (8)

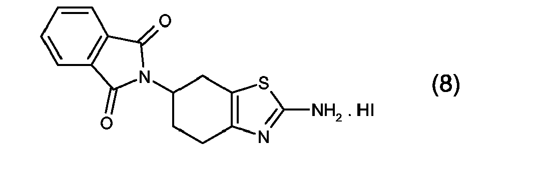

8. A process for the preparation of compound of formula (8) comprising step (b) as described in claims 1 to 3.

9. Use of a compound formula (8) for the manufacture of pramipexole.
